# EUROPEAN PATENT APPLICATION

(11) **EP 1 749 893 A2**
(43) Date of publication of application: **07.02.2007**
(21) Application number: 06021736.1
(22) Date of filing: 23.02.2001
(51) Int. Cl.: C12Q 1/68, C12N 15/00

(54) **Nucleic acid primers of acidophilic bacterium and method of identifying acidophilic bacterium**

(30) Priority: 14.03.2000 JP 2000070284
(62) Divisional of application: 01906234.8
(71) Applicant: OTSUKA PHARMACEUTICAL CO., LTD., Chiyoda-ku Tokyo 101-8535 (JP)
(72) Inventor: Takaichi, Akihisa, Naruto-shi, Tokushima 772-0051 (JP); Watanabe, Yoshinari, Itano-gun, Tokushima 771-1240 (JP); Okamoto, Toshihiko, Tokushima-shi, Tokushima 779-3122 (JP); Hanya, Izumi, Tokushima-shi, Tokushima 771-0130 (JP)
(74) Representative: HOFFMANN EITLE

(57) **Abstract**

The present invention provides a technique for rapidly and easily detecting and identifying a harmful acidophilic bacterium mixed in a beverage or like sample. The method comprises performing PCR using a primer pair of a specific combination of nucleic acid primers each comprising the nucleotide sequence shown in one of SEQ ID NOS: 4 to 8 or 11 to 14, to detect and identify an acidophilic bacterium in a sample.

## Description

### TECHNICAL FIELD

The present invention relates to nucleic acid primers having sequences specific to acidophilic bacteria, in particular acidophilic bacteria belonging to the genus *Alicyclobacillus,* and a method for identifying the acidophilic bacteria using the nucleic acid primers.

### BACKGROUND ART

It is difficult for most bacteria to grow in beverages with acidic properties, such as fruit juices and lactic acid bacteria beverages. Therefore, these beverages are free from the problems of contamination with those bacteria that have difficulty growing therein. However, the beverages may have serious problems of contamination by acidophilic bacteria capable of growing in acidic environments, in particular contamination by thermotolerant acidophilic bacteria that are difficult to kill by conventional heat sterilization processes.

There have been proposed several methods of testing for contamination by acidophilic bacteria that cause problems in these beverages, and several culture media for use in such methods. For example, Japanese Unexamined Patent Publication No. 1996-140697 discloses a selective medium for detecting a thermotolerant acidophilic bacterium and a method for detecting the bacterium. Japanese Unexamined Patent Publication No. 1996-140696 proposes a method for detecting a thermotolerant acidophilic bacterium that can grow in fruit juices.

In the proposed methods, the bacteria are detected by biochemical characteristic tests, such as analysis of the fatty acid composition of the bacterial cells. The property tests are disadvantageous in that they require much time and labor. Further, the detected cells are identified by general biological tests, which also involve a great deal of time and complicated steps and processes.

As a substitute for the above methods, a method has been recently proposed which quickly and easily detects the presence or absence of a specific acidophilic bacterium in a beverage sample by DNA analysis through PCR using part of a nucleotide sequence of a gene of the bacterium as a primer (Japanese Unexamined Patent Publication No. 1998-234376).

The primer used in this method has a part of a nucleic acid sequence coding for an enzyme possessed by a thermotolerant acidophilic bacterium and involved in the biosynthesis of ω-cyclohexane fatty acid. The method using this primer can detect only bacteria capable of producing an enzyme involved in the ω-cyclohexane fatty-acid biosynthesis. In other words, this method does not detect acidophilic bacteria but instead detects bacteria capable of producing a specific enzyme regardless of whether or not they are acidophilic. However, there is no direct relation between acidophilic bacteria and the ability to produce the specific enzyme. Thus, acidophilic bacteria that may contaminate beverages or the like do not necessarily have a common ability to produce the specific enzyme. Most acidophilic bacteria that cause contamination do not have the specific enzyme producing ability. Therefore, it is impossible to predict problems in food and beverages caused by contamination, such as an unpleasant odor, from the results of the detection by the above method.

The proposed method is therefore unsuitable for detecting an acidophilic bacterium contained in a beverage or like sample and liable to contaminate the sample. The method is incapable of confirming whether the beverage sample is safe or not (whether or not the sample is contaminated with a harmful acidophilic bacterium). Further, this method cannot identify the species of acidophilic bacteria.

An object of the present invention is to provide a method for selectively detecting and identifying an acidophilic bacterium.

### DISCLOSURE OF THE INVENTION

The present inventors carried out extensive research to achieve the above object. As a result, the inventors succeeded in synthesizing primers based on nucleotide sequences specific to genes of harmful acidophilic bacteria that may be contained in a beverage or like sample, and in developing rapid and easy methods for detecting and identifying harmful acidophilic bacteria in a sample by PCR using the primers. Thus, the inventors have accomplished the present invention.

The invention provides nucleic acid primers each comprising a nucleotide sequence shown in any one of SEQ ID NOS: 1 to 14.

The invention also provides the following primer pairs (1) to (8) for identifying acidophilic bacteria.
(1) A primer pair of a nucleic acid primer comprising the nucleotide sequence shown in SEQ ID NO: 1 and a nucleic acid primer comprising the nucleotide sequence shown in SEQ ID NO: 2.
(2) A primer pair of a nucleic acid primer comprising the nucleotide sequence shown in SEQ ID NO: 3 and a nucleic acid primer comprising the nucleotide sequence shown in SEQ ID NO: 2.
(3) A primer pair of a nucleic acid primer comprising the nucleotide sequence shown in SEQ ID NO: 4 and a nucleic acid primer comprising the nucleotide sequence shown in SEQ ID NO: 5.
(4) A primer pair of a nucleic acid primer comprising the nucleotide sequence shown in SEQ ID NO. 6 and a nucleic acid primer comprising the nucleotide sequence shown in SEQ ID NO: 7.
(5) A primer pair of a nucleic acid primer comprising the nucleotide sequence shown in SEQ ID NO: 6 and a nucleic acid primer comprising the nucleotide sequence shown in SEQ ID NO: 8.
(6) A primer pair of a nucleic acid primer comprising the nucleotide sequence shown in SEQ ID NO: 9 and a nucleic acid primer comprising the nucleotide sequence shown in SEQ ID NO: 10.
(7) A primer pair of a nucleic acid primer comprising the nucleotide sequence shown in SEQ ID NO: 11 and a nucleic acid primer comprising the nucleotide sequence shown in SEQ ID NO: 12.
(8) A primer pair of a nucleic acid primer comprising the nucleotide sequence shown in SEQ ID NO: 13 and a nucleic acid primer comprising the nucleotide sequence shown in SEQ ID NO: 14.

The invention further provides a method for identifying, through PCR using at least one of the nucleic acid primer pairs (1) to (8), at least one acidophilic bacterium, more specifically at least one acidophilic bacterium selected from the group consisting of *Alicyclobacillus acidocaldarius, Alicyclobacillus acidoterrestris* and *Alicyclobacillus cycloheptanicus* in a sample, especially in a beverage. The acidophilic bacterium identification method of the invention includes the following embodiments (a) to (c).
(a) An embodiment in which *Alicyclobacillus acidocaldarius* is identified using at least one of the primer pairs (1), (2) and (6).
(b) An embodiment in which *Alicyclobacillus acidoterrestris* is identified using at least one of the primer pairs (3) and (7).
(c) An embodiment in which *Alicyclobacillus cycloheptanicus* is identified using at least one of the primer pairs (4), (5) and (8).

In particular, the invention provides a method for carrying out the embodiments (a) to (c) by a single PCR reaction. This method can be achieved because the primer pairs of the invention are capable of amplifying targeted gene regions of each of the three species of acidophilic bacteria under the same PCR conditions. In this method, the three primer pairs of the invention specific to the three species of acidophilic bacteria are placed in, for example, wells of a multiwell plate, followed by PCR of a sample, so that the three species of acidophilic bacteria can be detected and identified simultaneously by a single PCR reaction. Thus, the method can easily and rapidly check for the presence of harmful acidophilic bacteria in the sample, and therefore can easily and quickly predict contamination of the sample.

The identification method of the invention tests whether the DNAs of the bacteria present in a sample hybridize the nucleic acid primers of the invention, to determine the presence or absence of acidophilic bacteria (specific microorganisms of the genus *Alicyclobacillus).*

In this specification, the abbreviations for amino acids, peptides, nucleotide sequences, nucleic acids, etc. are those provided in IUPAC-IUB Communication on Biological Nomenclature, Eur. J. Biochem., 138: 9 (1984) or "Guideline for preparation of a specification containing nucleotide sequences or amino acid sequences" (JPO), or those conventionally used in this field.

The following is detailed description of the nucleic acid primers, primer pairs and acidophilic bacterium identification method according to the invention.

It is essential for the nucleic acid primers of the invention to comprise one of the nucleotide sequences shown in SEQ ID NOS: 1 to 14. The nucleotide sequence shown in each sequence ID number basically corresponds to a part of the nucleotide sequence of an acidophilic bacterium 16S ribosomal RNA.

The present inventors directed their attention to the 16S ribosomal RNA genes of acidophilic bacteria, and found that a target acidophilic bacterium can be specifically identified by using a primer having the nucleotide sequence of a specific part of the 16S ribosomal RNA gene of the bacterium, or its homologous nucleotide sequence, namely a nucleotide sequence capable of hybridizing with the nucleotide sequence of the 16S ribosomal RNA gene under stringent conditions.

A 16S ribosomal RNA gene codes for a ribosomal RNA, which is a structural element of a ribosome (an important intracellular organelle for protein synthesis). The gene is present in all microorganisms.

The 16S ribosomal RNA genes of harmful acidophilic bacteria of the genus *Alicyclobacillus* that may be mixed in beverages are known per se. For example, regarding *Alicyclobacillus acidocaldarius* (ATCC27009), 1548 bp derived from the DSM446 strain registered in the EMBL/GenBank database under Accession Number X60742 is known [Int. J. Syst. Bacteriol., 42 (2), 263-269 (1992)]. Regarding *Alicyclobacillus acidoterrestris,* the 16S ribosomal RNA (1522 bp) of the deposited microorganism ATCC49025 shown in the doctoral thesis of Assistant Professor Yamazaki in Hokkaido University is known. Regarding *Alicyclobacillus cycloheptanicus* (ATCC49028), 1537 bp registered in the EMBL/GenBank database under Accession Number X51928 is known [Curr. Microbiol., 21, 325-327 (1990)].

Since the 16S ribosomal RNAs are species-specific, a particular partial sequence of the RNAs can be advantageously used for identification of a harmful acidophilic bacterium that may be present in a sample, such as a beverage.

The nucleic acid primers of the invention are designed so that the regions to be amplified have a length of about 100 to 2000 bp and the primers have a length of at least 10 mers, preferably about 13 to 30 mers, more preferably about 13 to 23 mers.

It is preferable that the two primers in a primer pair have the closest possible Tm values (Nakayama, Hiroki, "Shinpan Bio Jikken Illustrated: Honto ni fueru PCR (New Edition of Illustrated Biological Experiment: Efficient PCR)", Shujun-sha, page 25 (2nd ed., June 1, 1998), to improve the reproducibility of amplification. The nucleotide sequences of the primers are preferably as similar as possible to the nucleotide sequence of the gene, in order to improve specific binding properties to the gene region to be amplified. However, the nucleotide sequences of the primers need not be completely the same as the nucleotide sequence of the gene. A primer having one or several bases different from those in the gene can amplify a desired nucleotide sequence of the gene to detect a specific acidophilic bacterium. Examples of such primers include those comprising a nucleotide sequence shown in SEQ ID NO: 3 or 8.

The primers of the invention can be easily synthesized by a known method, considering the above conditions. For example, the primers can be chemically synthesized by the phosphoramidite method or triester method. Also, the primers can be synthesized by the β-cyanoethyl synthesis method using a commercial automatic oligonucleotide synthesizer, for example, an automatic DNA synthesizer available from Perkin-Elmer or other manufacturers.

A double-stranded fragment can be obtained in the following manner, using a chemically synthesized single-stranded product. First, a complementary strand of the single-stranded product was synthesized, and then, under suitable conditions, the complementary strand is annealed with the single-stranded product or added to the single-stranded product using a suitable primer sequence and DNA polymerase.

The primer of the invention thus obtained can be defined as a DNA that hybridizes, under stringent conditions, with a DNA comprising the nucleotide sequence of the 16S ribosomal RNA of a specific acidophilic bacterium. The "stringent conditions" may be conditions under which a primer is usually used. Examples of such conditions are: at 50°C in 0.2 x SSC containing 0.1% SDS, and at 60°C in 1 x SSC containing 0.1% SDS.

Specific examples of the nucleotide sequences of the nucleic acid primers of the invention include those shown in SEQ ID NOS: 1 to 14. The nucleotide sequence of SEQ ID NO: 1 corresponds to the nucleotides from 161 to 180 of the nucleotide sequence of the 16S ribosomal RNA gene of *Alicyclobacillus acidocaldarius* DSM446 (Accession Number X60742, 1548 bp). The sequence of SEQ ID NO: 3 is the same as the above sequence except that the 3rd A is substituted by G.

The nucleotide sequence of SEQ ID NO: 2 corresponds to the complementary strand sequence of the nucleotides from 591 to 611 of the nucleotide sequence of the 16S ribosomal RNA gene of *Alicyclobacillus acidocaldarius* DSM446 (Accession Number X60742, 1548 bp).

The nucleotide sequence of SEQ ID NO: 4 corresponds to the nucleotides from 172 to 192 of the nucleotide sequence of the 16S ribosomal RNA gene (1522 bp) of *Alicyclobacillus acidoterrestris* ATCC49025.

The nucleotide sequence of SEQ ID NO: 5 corresponds to the nucleotides from 587 to 609 of the nucleotide sequence of the 16S ribosomal RNA gene (1522 bp) of *Alicyclobacillus acidoterrestris* ATCC49025.

The nucleotide sequence of SEQ ID NO: 6 corresponds to the nucleotides from 186 to 207 of the nucleotide sequence of the 16S ribosomal RNA gene of *Alicyclobacillus cycloheptanicus* (Accession Number X51928, 1537 bp).

The nucleotide sequence of SEQ ID NO: 7 corresponds to the nucleotides from 583 to 603 of the nucleotide sequence of the 16S ribosomal RNA gene of *Alicyclobacillus cycloheptanicus* (Accession Number X51928, 1537 bp).

SEQ ID NO: 8 is the same as SEQ ID NO: 7 except that the 11th to 14th cccg is substituted by ttc.

The nucleotide sequence of SEQ ID NO: 9 corresponds to the nucleotides from 168 to 180 of the nucleotide sequence of the 16S ribosomal RNA gene of *Alicyclobacillus acidocaldarius* (Accession Number X60742, 1548 bp).

The nucleotide sequence of SEQ ID NO: 10 corresponds to the complementary strand sequence of the nucleotides from 591 to 605 of the nucleotide sequence of the 16S ribosomal RNA gene of *Alicyclobacillus acidocaldarius* (Accession Number X60742, 1548 bp).

The nucleotide sequence of SEQ ID NO: 11 corresponds to the nucleotides from 176 to 192 of the nucleotide sequence of the 16S ribosomal RNA gene of *Alicyclobacillus acidoterrestris* ATCC49025 (1522 bp).

The nucleotide sequence of SEQ ID NO: 12 corresponds to the nucleotides from 587 to 606 of the nucleotide sequence of the 16S ribosomal RNA gene of *Alicyclobacillus acidoterrestris* ATCC49025 (1522 bp).

The nucleotide sequence of SEQ ID NO: 13 corresponds to the nucleotides from 191 to 207 of the nucleotide sequence of the 16S ribosomal RNA gene of *Alicyclobacillus cycloheptanicus* (Accession Number X51928, 1537 bp).

The nucleotide sequence of SEQ ID NO: 14 corresponds to the nucleotides from 583 to 595 of the nucleotide sequence of the 16S ribosomal RNA gene of *Alicyclobacillus cycloheptanicus* (Accession Number X51928, 1537 bp).

Examples of combinations of the above primers (primer pairs of a forward primer and a reverse primer) usable in the method of the invention include the above primer pairs (1) to (8).

The use of the primer pairs of the invention enables detection and identification of acidophilic bacteria *(Alicyclobacillus acidocaldarius, Alicyclobacillus acidoterrestris* and *Alicyclobacillus cycloheptanicus*) by confirming the presence or absence of amplified DNA in a sample.

A typical example of the sample for use in the method of the invention is a beverage. Other examples of the sample include soils, vegetables, meats, confections, etc. Preferably, test bacteria are separated from the sample by fishing from colonies cultured in a suitable medium.

According to the method of the invention, the test bacteria separated in the above manner are first subjected to DNA extraction by a known process, such as phenol extraction, to obtain a DNA sample. A primer pair of the invention and DNA polymerase are made to act on a part of the DNA sample under specific conditions to perform PCR. The PCR amplifies a specific gene region of the DNA sample. Thus, the resulting PCR mixture is subjected, for example, to electrophoresis, to separate the amplified DNAs by size and confirm the presence of DNA amplification products.

In this manner, the method of the invention is capable of determining whether the sample contains an acidophilic bacterium, by checking for the presence of a DNA amplification product of the expected size. Further, the nucleotide sequence of the DNA amplification product may be determined and compared with the nucleotide sequence of the gene region of a specific acidophilic bacterium, to accomplish more reliable detection and identification.

The PCR can be performed by a conventional method (see, for example, Science, 230, 1350 (1985)).

The amplified DNA fragment can be separated and purified by a conventional method, such as gel electrophoresis.

The nucleotide sequences of the primers of the invention and the separated and purified DNA fragment can also be determined by a conventional method, for example, the dideoxy method [Proc. Natl. Acad. Sci., USA., 74, 5463 (1977)], the Maxam-Gilbert method [Methods in Enzymology, 65, 499 (1980)] or like method. A commercial sequence kit or the like can be employed to facilitate the determination of the nucleotide sequences.

The primers of the invention can be used as probes for a DNA hybridization method to identify acidophilic bacteria. This method is carried out, for example, as follows: A DNA sample prepared from the sample in the above manner is adsorbed and immobilized on a polyamide membrane or the like, and hybridized with a probe labeled with biotin or the like. In this method, if the DNA sample is complementary to the probe, a double strand is formed on the membrane or the like. The acidophilic bacterium to be detected can be identified by measuring the activity of the label in the double strand.

### BRIEF DESCRIPTION OF THE DRAWINGS

Fig. 1 is a drawing showing the results of electrophoresis of PCR products in an acidophilic bacterium identification test using primer pairs of the invention.
Fig. 2 is a drawing showing the results of electrophoresis of PCR products in an acidophilic bacterium identification test using primer pairs of the invention.
Fig. 3 is a drawing showing the results of electrophoresis of PCR products in an acidophilic bacterium identification test using primer pairs of the invention.

### BEST MODE FOR CARRYING OUT THE INVENTION

Examples are given below to illustrate the invention in further detail.

### Example 1

### (1) Test bacteria

The deposited bacteria shown in Table 1 were used as test bacteria.

**Table 1**

| Species | Strain |
|---|---|
| *A. acidocaldarius* | IFO15652 (type strain) |
| *A. acidoterrestris* | ATCC49025 (type strain) |
| *A. cycloheptanicus* | IFO15310 (type strain) |
| *B. subtilis* | ATCC6633 |
| *B. coagulans* | IAM1115 (type strain) |
| *B. stearothermophilus* | IAM1035 |
| *Cl. sporogenes* | IAM19235 |

### (2) Preparation of DNA solutions

A DNA solution was prepared from each test bacterium using InstaGene DNA purification matrix (Bio-Rad), according to the company's manual. Specifically, bacterial cells was subjected to a washing procedure comprising suspending a loop of the cells in 800 µl of sterilized water, centrifuging the suspension (10000 G, 10 minutes), and removing the supernatant to separate the precipitate (cells). This washing procedure was repeated once again. InstaGene DNA purification matrix (200 µl) was added to the obtained precipitate (cells), followed by incubation at 56 °C for 30 minutes. Then, the cells were dissolved by stirring (using Vortex) and boiling (8 minutes), to thereby obtain a DNA solution.

### (3) Design and synthesis of the primers of the invention

The primers of the invention shown in Table 2 were designed and synthesized from the nucleotide sequences of the 16S ribosomal RNA gene of *Alicyclobacillus acidocaldarius* (Accession Number X60742 (EMBL/GenBank database) [Int. J. Syst. Bacteriol., 42 (2), 263-269 (1992)], the 16S ribosomal RNA gene of *Alicyclobacillus acidoterrestris* (the doctoral thesis by Assistant Professor Yamazaki in Hokkaido University), and the 16S ribosomal RNA gene of *Alicyclobacillus cycloheptanicus* (Accession Number X51928 (EMBL/GenBank database) [Curr. Microbiol., 21, 325-327 (1990)]. An automatic DNA synthesizer (Perkin-Elmer) was used for the synthesis.

**Table 2**

| Species | Primer | Nucleotide sequence | Tm (°C) |
|---|---|---|---|
| *A. acidocaldarius* | Forward primer | SEQ ID NO: 1 | 61.6 |
| *A. acidocaldarius* | Reverse primer | SEQ ID NO: 2 | 57.6 |
| *A. acidocaldarius* | Forward primer | SEQ ID NO: 3 | 63.6 |
| *A. acidoterrestris* | Forward primer | SEQ ID NO: 4 | 57.6 |
| *A. acidoterrestris* | Reverse primer | SEQ ID NO: 5 | 54.2 |
| *A. cycloheptanicus* | Forward primer | SEQ ID NO: 6 | 59.5 |
| *A. cycloheptanicus* | Reverse primer | SEQ ID NO: 7 | 63.3 |
| *A. cycloheptanicus* | Reverse primer | SEQ ID NO: 8 | 57.6 |

### (4) Identification of acidophilic bacteria by PCR

### (4-1) Preparation of PCR mixtures

According to the formulation shown in Table 3, reagents and each DNA solution prepared in (2) were placed in a 0.2 ml tube (TAKARA SHUZO CO., LTD.) to prepare PCR mixtures. This procedure was carried out on ice.

**Table 3**

| Component | Amount (µl/25 µl) | Final concentration |
|---|---|---|
| 5 U/µl TaKaRa Taq | 0.25 | 1.25 U/25 µl |
| 10 x PCR buffer (MgCl₂-free) | 2.5 | Onefold concentration |
| 25 mM MgCl₂ | 2 | 2 mM |
| 2.5 mM dNTP mixture | 2 | 200 µM |
| 4 pmol/µl forward primer | 2 | 320 nM |
| 4 pmol/µl reverse primer | 2 | 320 nM |
| DNA template | 1 | - |
| Sterilized distilled water | 13.25 | - |
| Total | 25 | - |

### (4-2) PCR

Each of the tubes containing the mixtures prepared in (4-1) was set in the heat block of a thermal cycler (TaKaRa PCR Thermal Cycler MP, TAKARA SHUZO CO., LTD.). Then, PCR was performed under the temperature conditions shown in Table 4. Specifically, after carrying out thermal denaturation at 94°C for 4 minutes, a cycle consisting of thermal denaturation at 94°C for 30 seconds, annealing at 60°C for 30 seconds and an extension reaction at 72°C for 1 minute, was performed 29 times. Finally, thermal denaturation at 94°C for 30 seconds, annealing at 60°C for 30 seconds and an extension reaction at 72°C for 5 minutes were conducted.

**Table 4**

| Step | Temperature (°C) | Cycle 1 | Cycle 2 | Cycle 3 |
|---|---|---|---|---|
| | | Time | Time | Time |
| Thermal denaturation | 94 | 4 min. | 30 sec. | 30 sec. |
| Annealing | 60 | - | 30 sec. | 30 sec. |
| Extension reaction | 72 | - | 1 min. | 5 min. |
| Number of cycle(s) | | 1 | 29 | 1 |

The primer pairs used were: (A) a combination of the primer of SEQ ID NO: 1 and the primer of SEQ ID NO: 2; (B) a combination of the primer of SEQ ID NO: 3 and the primer of SEQ ID NO: 2; (C) a combination of the primer of SEQ ID NO: 4 and the primer of SEQ ID NO: 5; (D) a combination of the primer of SEQ ID NO: 6 and the primer of SEQ ID NO: 7; and (E) a combination of the primer of SEQ ID NO: 6 and the primer of SEQ ID NO: 8. The first test was carried out using the primer pairs (A), (C) and (D), and the second test was performed using the primer pairs (B), (C) and (E).

### (4-3) Electrophoresis

After completion of the PCR, 5 µl of 6 x Loading buffer (TAKARA SHUZO CO., LTD.) was added to 25 µl of the reaction mixture. Then, 6 µl of the resulting mixture was subjected to electrophoresis in a 1.5% agarose gel (Agarose LO3 TAKARA SHUZO CO., LTD., 1 x TBE buffer) (electrophoresis equipment: Mupid, Cosmo Bio Co.).

### (4-4) Observation

After the electrophoresis, the gel was dyed with 1 µg/ml of ethidium bromide for 20 minutes and washed with water for 10 minutes. The electrophoresis pattern of the gel was observed under ultraviolet rays (Mini-Transiluminator, Bio-Rad), and a Polaroid photograph of the pattern was taken.

### (4-5) Results

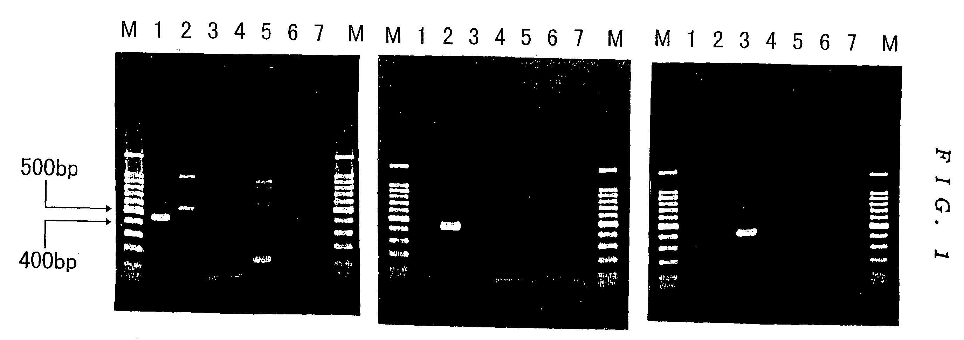

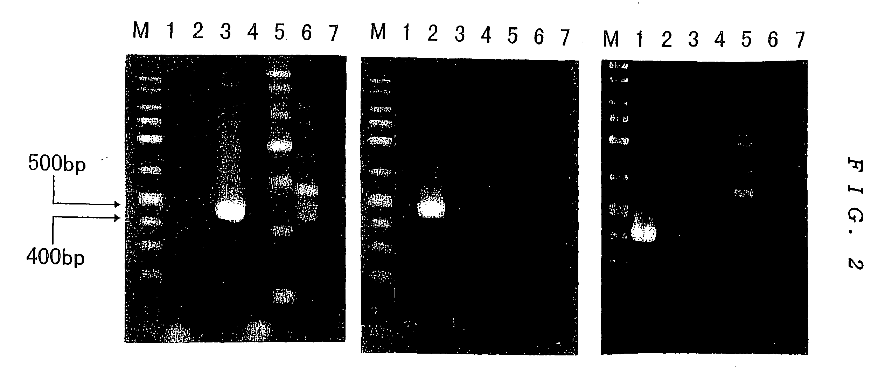

Figs. 1 and 2 show the results of electrophoresis (photographs).

Fig. 1 presents the results of the first test (using the primer pairs (A), (C) and (D)). Fig. 2 presents the results of the second test (using the primer pairs (B), (C) and (E)). The lanes in Figs. 1 and 2 indicate the following.

### Fig. 1:

- Lane M: Molecular weight marker
- Lane 1: *A. acidocaldarius*
- Lane 2: *A. acidoterrestris*
- Lane 3: *A. cycloheptanicus*
- Lane 4: *B. subtilis*
- Lane 5: *B. coagulans*
- Lane 6: *B. stearothermophilus*
- Lane 7: *Cl. sporogenes*

### Fig. 2:

- Lane M: Molecular weight marker
- Lane 1: *A. cycloheptanicus*
- Lane 2: *A. acidoterrestris*
- Lane 3: *A. acidocaldarius*
- Lane 4: *B. subtilis*
- Lane 5: *B. coagulans*
- Lane 6: *B. stearothermophilus*
- Lane 7: *Cl. sporogenes*

Based on the results shown in the photographs, the relationship between the use of each primer pair and detectability of each test bacterium was investigated (+; detected, -; not detected). Table 5 presents the results.

**Table 5**

| Bacterial species | Primer pair | | | | | |
|---|---|---|---|---|---|---|
| | (A) | (C) | (D) | (B) | (C) | (E) |
| *A. acidocaldarius* | + | - | - | + | - | - |
| *A. acidoterrestris* | - | + | - | - | + | - |
| *A. cycloheptanicus* | - | - | + | - | - | + |
| *B. subtilis* | - | - | - | - | - | - |
| *B. coagulans* | - | - | - | - | - | - |
| *B. stearothermophilus* | - | - | - | - | - | - |
| Cl. sporogenes | - | - | - | - | - | - |

The results presented in Fig. 1, Fig. 2 and Table 5 reveal that when the test bacteria were the three species, *Alicyclobacillus acidocaldarius, Alicyclobacillus acidoterrestris* and *Alicyclobacillus cycloheptanicus,* PCR products of the targeted lengths of about 450 bp, 430 bp and 400 bp, respectively, were formed by the use of the primer pairs of the invention. On the other hand, it was confirmed that no PCR product was formed when the test bacteria were other similar species.

This demonstrates that the use of one of the primer pairs of the invention enables reliable detection and identification of one of the three species of acidophilic bacteria.

The above tests were performed under the same PCR conditions. Therefore, when three types of primer pairs of the invention specific to the three species of acidophilic bacteria, respectively, were used in combination, the three species can be detected and identified by a single PCR. Thus, the method of the invention is capable of selectively, easily and rapidly identifying harmful acidophilic bacteria that may be present in a sample. It is therefore possible to predict contamination of the sample with the acidophilic bacteria.

### Example 2

### (1) Test bacteria

The deposited bacteria shown in Table 1 were used as test bacteria.

### (2) Preparation of DNA solutions

DNA solutions were prepared in the same manner as in (2) in Example 1.

### (3) Design and synthesis of the primers of the invention

The primers of the invention shown in Table 6 were designed and synthesized from the nucleotide sequences of the 16S ribosomal RNA gene of *Alicyclobacillus acidocaldarius* (Accession Number X60742 (EMBL/GenBank database) [Int.J.Syst.Bacteriol., 42 (2), 263-269 (1992)], the 16S ribosomal RNA gene of *Alicyclobacillus acidoterrestris* (the doctoral thesis by Assistant Professor Yamazaki in Hokkaido University) and the 16S ribosomal RNA gene of *Alicyclobacillus cycloheptanicus* (Accession Number X51928 (EMBL/GenBank database) [Curr. Microbiol. 21, 325-327 (1990)]. An automatic DNA synthesizer (Perkin-Elmer) was used for the synthesis.

**Table 6**

| Species | Primer | Nucleotide sequence | Tm (°C) |
|---|---|---|---|
| *A. acidocaldarius* | Forward primer | SEQ ID NO: 9 | 53.0 |
| *A. acidocaldarius* | Reverse primer | SEQ ID NO: 10 | 51.2 |
| *A. acidoterrestris* | Forward primer | SEQ ID NO: 11 | 49.8 |
| *A. acidoterrestris* | Reverse primer | SEQ ID NO: 12 | 49.3 |
| *A. cycloheptanicus* | Forward primer | SEQ ID NO: 13 | 49.8 |
| *A. cycloheptanicus* | Reverse primer | SEQ ID NO: 14 | 53.0 |

### (4) Identification of acidophilic bacteria by PCR

### (4-1) Preparation of PCR mixtures

According to the formulation shown in Table 3, reagents and each DNA solution prepared in (2) were placed in a 0.2 ml tube (TAKARA SHUZO CO., LTD.) to prepare PCR mixtures. The above procedure was carried out on ice.

### (4-2) PCR

Each of the tubes containing the mixtures prepared in (4-1) was set in the heat block of a thermal cycler (TaKaRa PCR Thermal Cycler MP, TAKARA SHUZO CO., LTD.). Then, PCR was performed under the temperature conditions described in Table 4.

The primer pairs used were: (F) a combination of the primer of SEQ ID NO: 9 and the primer of SEQ ID NO: 10; (G) a combination of the primer of SEQ ID NO: 11 and the primer of SEQ ID NO: 12; and (H) a combination of the primer of SEQ ID NO: 13 and the primer of SEQ ID NO: 14. (4-3) Electrophoresis and observation

After completion of the PCR, 5 µl of 6 x Loading buffer (TAKARA SHUZO CO., LTD.) was added to 25 µl of the reaction mixture. Then, 6 µl of the resulting mixture was subjected to electrophoresis in a 1.5% agarose gel (Agarose L03, TAKARA SHUZO CO., LTD., 1 x TBE buffer) (electrophoresis equipment: Mupid, Cosmo Bio Co.).

After the electrophoresis, the gel was dyed with 1 µg/ml of ethidium bromide for 20 minutes and washed with water for 10 minutes. The migration pattern of the gel was observed under ultraviolet rays (Mini-Transiluminator, Bio-Rad), and a Polaroid photograph of the pattern was taken.

### (5) Results

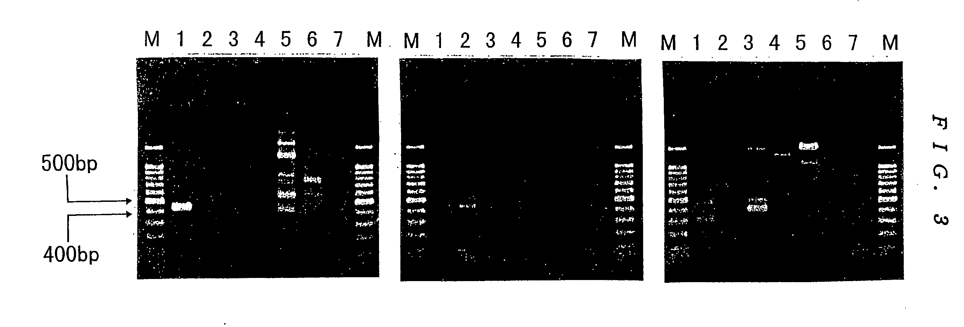

Fig. 3 shows the results of the electrophoresis (photographs).

Fig. 3 shows, from left to right, the results of using the primer pairs (F), (G) and (H). The lanes are the same as those in Fig. 1.

Based on the results shown in the photographs, the relationship between the use of each primer pair and the detectability of each test bacterium was investigated (+; detected, -; not detected). Table 7 presents the results.

**Table 7**

| Bacterial species | Primer pair | | |
|---|---|---|---|
| | (F) | (G) | (H) |
| *A. acidocaldarius* | + | - | - |
| *A. acidoterrestris* | - | + | - |
| *A. cycloheptanicus* | - | - | + |
| *B. subtilis* | - | - | - |
| *B. coagulans* | - | - | - |
| *B. stearothermophilus* | - | - | - |
| *Cl. sporogenes* | - | - | - |

The results shown in Fig. 3 and Table 7 reveal that when the test bacteria were the three species, i.e., *Alicyclobacillus acidocaldarius, Alicyclobacillus acidoterrestris* and *Alicyclobacillus cycloheptanicus,* PCR products of the desired lengths of about 450 bp, 430 bp and 400 bp, respectively, were formed by the use of the primer pairs of the invention. On the other hand, it was confirmed that no PCR product was formed when the test bacteria were other similar species. This demonstrates that the use of the primers of the invention enables reliable detection and identification of the three species of acidophilic bacteria.

The three species of acidophilic bacteria can be detected and identified simultaneously by a single PCR reaction, when performing the above test using, as a sample, a beverage or the like that may be contaminated with the acidophilic bacteria. For example, the primer pairs of the invention each specific to one of the three species are individually placed in wells of a multiwell plate, and the sample is subjected to PCR. Accordingly, the invention is capable of easily and rapidly determining contamination of the sample with the acidophilic bacteria.

### INDUSTRIAL APPLICABILITY

The present invention provides nucleic acid primers specific to acidophilic bacteria. The use of the primers of the invention makes it possible to selectively, easily and rapidly detect and identify acidophilic bacteria that cause contamination of a beverage or like sample.

## Claims

1. A nucleic acid primer comprising the nucleotide sequence shown in any one of SEQ ID NO: 4 to 8 and 11 to 14.

2. A nucleic acid primer comprising the nucleotide sequence shown in SEQ ID NO: 4.

3. A nucleic acid primer comprising the nucleotide sequence shown in SEQ ID NO: 5.

4. A nucleic acid primer comprising the nucleotide sequence shown in SEQ ID NO: 6.

5. A nucleic acid primer comprising the nucleotide sequence shown in SEQ ID NO: 7.

6. A nucleic acid primer comprising the nucleotide sequence shown in SEQ ID NO: 8.

7. A nucleic acid primer comprising the nucleotide sequence shown in SEQ ID NO: 11.

8. A nucleic acid primer comprising the nucleotide sequence shown in SEQ ID NO: 12.

9. A nucleic acid primer comprising the nucleotide sequence shown in SEQ ID NO: 13.

10. A nucleic acid primer comprising the nucleotide sequence shown in SEQ ID NO: 14.

11. A primer pair for identifying an acidophilic bacterium, which is any one of the following primer pairs: (3) a primer pair of a nucleic acid primer comprising the nucleotide sequence shown in SEQ ID NO: 4 and a nucleic acid primer comprising the nucleotide sequence shown in SEQ ID NO: 5; (4) a primer pair of a nucleic acid primer comprising the nucleotide sequence shown in SEQ ID NO: 6 and a nucleic acid primer comprising the nucleotide sequence shown in SEQ ID NO: 7; (5) a primer pair of a nucleic acid primer comprising the nucleotide sequence shown in SEQ ID NO: 6 and a nucleic acid primer comprising the nucleotide sequence shown in SEQ ID NO: 8; (7) a primer pair of a nucleic acid primer comprising the nucleotide sequence shown in SEQ ID NO: 11 and a nucleic acid primer comprising the nucleotide sequence shown in SEQ ID NO: 12; (8) a primer pair of a nucleic acid primer comprising the nucleotide sequence shown in SEQ ID NO: 13 and a nucleic acid primer comprising the nucleotide sequence shown in SEQ ID NO: 14.

12. A primer pair according to Claim 11, which is a primer pair of a nucleic acid primer comprising the nucleotide sequence shown in SEQ ID NO: 4 and a nucleic acid primer comprising the nucleotide sequence shown in SEQ ID NO: 5.

13. A primer pair according to Claim 11, which is a primer pair of a nucleic acid primer comprising the nucleotide sequence shown in SEQ ID NO: 6 and a nucleic acid primer comprising the nucleotide sequence shown in SEQ ID NO: 7.

14. A primer pair according to Claim 11, which is a primer pair of a nucleic acid primer comprising the nucleotide sequence shown in SEQ ID NO: 6 and a nucleic acid primer comprising the nucleotide sequence shown in SEQ ID NO: 8.

15. A primer pair according to Claim 11, which is a primer pair of a nucleic acid primer comprising the nucleotide sequence shown in SEQ ID NO: 11 and a nucleic acid primer comprising the nucleotide sequence shown in SEQ ID NO: 12.

16. A primer pair according to Claim 11, which is a primer pair of a nucleic acid primer comprising the nucleotide sequence shown in SEQ ID NO: 13 and a nucleic acid primer comprising the nucleotide sequence shown in SEQ ID NO: 14.

17. A method for identifying at least one acidophilic bacterium present in a sample, comprising performing PCR using at least one primer pair selected from the group consisting of: (3) a primer pair of a nucleic acid primer comprising the nucleotide sequence shown in SEQ ID NO: 4 and a nucleic acid primer comprising the nucleotide sequence shown in SEQ ID NO: 5; (4) a primer pair of a nucleic acid primer comprising the nucleotide sequence shown in SEQ ID NO: 6 and a nucleic acid primer comprising the nucleotide sequence shown in SEQ ID NO: 7; (5) a primer pair of a nucleic acid primer comprising the nucleotide sequence shown in SEQ ID NO: 6 and a nucleic acid primer comprising the nucleotide sequence shown in SEQ ID NO: 8; (7) a primer pair of a nucleic acid primer comprising the nucleotide sequence shown in SEQ ID NO: 11 and a nucleic acid primer comprising the nucleotide sequence shown in SEQ ID NO: 12; and (8) a primer pair of a nucleic acid primer comprising the nucleotide sequence shown in SEQ ID NO: 13 and a nucleic acid primer comprising the nucleotide sequence shown in SEQ ID NO: 14.

18. A method according to Claim 17, wherein the acidophilic bacterium is Alicyclobacillus acidoterrestris and the primer pair is at least one member selected from the group consisting of: (3) a primer pair of a nucleic acid primer comprising the nucleotide sequence shown in SEQ ID NO: 4 and a nucleic acid primer comprising the nucleotide sequence shown in SEQ ID NO: 5; and (7) a primer pair of a nucleic acid primer comprising the nucleotide sequence shown in SEQ ID NO: 11 and a nucleic acid primer comprising the nucleotide sequence shown in SEQ ID NO: 12.

19. A method according to Claim 17, wherein the acidophilic bacterium is Alicyclobacillus cycloheptanicus, and the primer pair is at least one member selected from the group consisting of: (4) a primer pair of a nucleic acid primer comprising the nucleotide sequence shown in SEQ ID NO: 6 and a nucleic acid primer comprising the nucleotide sequence shown in SEQ ID NO: 7; (5) a primer pair of a nucleic acid primer comprising the nucleotide sequence shown in SEQ ID NO: 6 and a nucleic acid primer comprising the nucleotide sequence shown in SEQ ID NO: 8; and (8) a primer pair of a nucleic acid primer comprising the nucleotide sequence shown in SEQ ID NO: 13 and a nucleic acid primer comprising the nucleotide sequence shown in SEQ ID NO: 14.

20. A method according to Claim 17, wherein the acidophilic bacterium is at least one species selected from the group consisting of Alicyclobacillus acidoterrestris and Alicyclobacillus cycloheptanicus, and the primer pair is at least one member selected from Group 2, and at least one member selected from Group 3: Group 2: (3) a primer pair of a nucleic acid primer comprising the nucleotide sequence shown in SEQ ID NO: 4 and a nucleic acid primer comprising the nucleotide sequence shown in SEQ ID NO: 5; and (7) a primer pair of a nucleic acid primer comprising the nucleotide sequence shown in SEQ ID NO: 11 and a nucleic acid primer comprising the nucleotide sequence shown in SEQ ID NO: 12; Group 3: (4) a primer pair of a nucleic acid primer comprising the nucleotide sequence shown in SEQ ID NO: 6 and a nucleic acid primer comprising the nucleotide sequence shown in SEQ ID NO: 7; (5) a primer pair of a nucleic acid primer comprising the nucleotide sequence shown in SEQ ID NO: 6 and a nucleic acid primer comprising the nucleotide sequence shown in SEQ ID NO: 8; and (8) a primer pair of a nucleic acid primer comprising the nucleotide sequence shown in SEQ ID NO: 13 and a nucleic acid primer comprising the nucleotide sequence shown in SEQ ID NO: 14.

21. A method according to Claim 17, wherein the sample is a beverage.


SEQUENCE LISTING

<110> Otsuka Pharmaceutical Co., Ltd.

<120> Primer of acidophile bacterium and method for detection of the

microorganisms

<130> P01-09

<150> JP 2000-70284

<151> 2000-03-14

<160> 14

<170> PatentIn Ver. 2.0

<210> 1

<211> 20

<212> DNA

<213> Artificial Sequence

<223> primer for A. acidocaldarius

<400> 1

ctaatgccgg atacgcccgc 20

<210> 2

<211> 21

<212> DNA

<213> Artificial Sequence

<223> primer for A. acidocaldarius

<400> 2

ctttcactcc agacttgctc g                                                    20

<210> 3

<211> 20

<212> DNA

<213> Artificial Sequence

<223> primer for A. acidocaldarius

<400> 3

ctgatgccgg atacgcccgc                                                      20

<210> 4

<211> 21

<212> DNA

<213> Artificial Sequence

<223> primer for A. acidoterrestris

<400> 4

acgggtaggc atctacttgt g                                                    21

<210> 5

<211> 23

<212> DNA

<213> Artificial Sequence

<223> primer for A. acidoterrestris

<400> 5

tggactttca cttcagactt aca                                                23

<210> 6

<211> 22

<212> DNA

<213> Artificial Sequence

<223> primer for A. cycloheptanicus

<400> 6

cgctgggaaa ggtgcaaatg ca                                                 22

<210> 7

<211> 22

<212> DNA

<213> Artificial Sequence

<223> primer for A. cycloheptanicus

<400> 7

ggactttcac cccggacaca cg                                                 22

<210> 8

<211> 21

<212> DNA

<213> Artificial Sequence

<223> primer for A. cycloheptanicus

<400> 8

ggactttcac ttcgacacac g 21

<210> 9

<211> 13

<212> DNA

<213> Artificial Sequence

<223> primer for A. acidocaldarius

<400> 9

cggatacgcc cgc 13

<210> 10

<211> 15

<212> DNA

<213> Artificial Sequence

<223> primer for A. acidocaldarius

<400> 10

ctccagactt gctcg                                                                15

<210> 11

<211> 17

<212> DNA

<213> Artificial Sequence

<223> primer for A. acidoterrestris

<400> 11

gtaggcatct acttgtg                                                              17

<210> 12

<211> 20

<212> DNA

<213> Artificial Sequence

<223> primer for A. acidoterrestris

<400> 12

actttcactt cagacttaca                                                           20

<210> 13

<211> 17

<212> DNA

<213> Artificial Sequence

<223> primer for A. cycloheptanicus

<400> 13

ggaaaggtgc aaatgca                                                    17

<210> 14

<211> 13

<212> DNA

<213> Artificial Sequence

<223> primer for A. cycloheptanicus

<400> 14

ccccggacac acg                                                        13